(19) Europäisches Patentamt
European Patent Office
Office européen des brevets

(11) Publication number: 0 079 390
B1

(12) EUROPEAN PATENT SPECIFICATION

(45) Date of publication of patent specification: 27.12.85

(51) Int. Cl.$^4$: C 07 C 101/30

(21) Application number: 82901531.2

(22) Date of filing: 19.05.82

(86) International application number:
PCT/JP82/00182

(87) International publication number:
WO 82/04044 25.11.82 Gazette 82/28

# (54) PROCESS FOR PREPARING BETA-HYDROXY AMINO ACID.

(30) Priority: 19.05.81 JP 74287/81
25.05.81 JP 78134/81

(43) Date of publication of application:
25.05.83 Bulletin 83/21

(45) Publication of the grant of the patent:
27.12.85 Bulletin 85/52

(84) Designated Contracting States:
CH DE FR GB LI NL

(56) References cited:
EP-A-0 030 474

Chemical Abstracts, Vol. 54, No. 21, 10. Nov. 1960 (10.11.60), (Columbus, Ohio, U.S.A.); K.D. Gunderman, G. Holtzmann, H.J. Rose and H. Schulze: "Formation, ring cleavage, and isomerization of ethyleneimine - 2 - carboxylic acid derivatives", pp. 1632-43, abstract No. 22552i

(73) Proprietor: MITSUI TOATSU CHEMICALS, INCORPORATED
2-5 Kasumigaseki 3-chome
Chiyoda-Ku Tokyo 100 (JP)

(72) Inventor: KAWASHIMA, Nobuyuki
4-1-28, Hase, Kamakura-shi
Kanagawa 248 (JP)
Inventor: KATOH, Toshio
600-1, Kamisakunobe, Takatsu-ku
Kawasaki-shi Kanagawa 213 (JP)
Inventor: MITA, Ryuichi
529, Shimosakunobe Takatsu-ku
Kawasaki-shi Kanagawa 213 (JP)
Inventor: OHOKA, Masaharu
2092, Iijimacho, Totsuka-ku
Yokohama-shi Kanagawa 244 (JP)
Inventor: HIGUCHI, Chojiro
4-5-13, Dai, Kamakura-shi
Kanagawa 247 (JP)
Inventor: KAWASHIMA, Nobuhiro
8587-3, Tana, Sagamihara-shi
Kanagawa 229 (JP)
Inventor: YAMAGUCHI, Akihiro
1-1-21, Iwase, Kamakura-shi
Kanagawa 247 (JP)

EP 0 079 390 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

0 079 390

(72) Inventor: **NAGAI, Shousuke**
**1071-2, Nakano-cho, Totsuka-ku**
**Yokohama-shi Kanagawa 247 (JP)**
Inventor: **TAKANO, Takao**
**380-172, Watauchi**
**Fujisawa-shi Kanagawa 251 (JP)**

(74) Representative: **Schüler, Horst, Dr.**
**European Patent Attorney**
**Kaiserstrasse 41**
**D-6000 Frankfurt/Main 1 (DE)**

**Description**

Technical Field

This invention relates to a process for producing a β-hydroxyamino acid represented by the general formula (I):

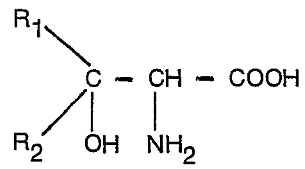

(I)

wherein $R_1$ represents a hydrogen atom, a methyl group, or a substituted or unsubstituted phenyl or pyridyl group and $R_2$ means a hydrogen atom or a methyl group.

Background Art

Serine, an example of β-hydroxyamino acids, is an α-amino acid and its optically active isomer, L-serine, is useful to prepare an amino acid solution for infusion. The other optical isomer, D-serine, is also a valuable compound as a raw material for antibiotic cycloserine. Furthermore, serine has also found utility as a raw material for L-tryptophan which is expected to have an increasing commercial utility as an additive to livestock feed.

As exemplary β-substituted-β-hydroxyamino acids, may be mentioned threonine, phenylserine, β-hydroxyvaline and the like. Threonine is one of the essential amino acids and has a promising future as an additive to livestock feed. β-substituted-β-hydroxyamino acids, led by phenylserine and β-hydroxyvaline, are finding increasing commercial utility owing to their own physiological activities and are also useful compounds as intermediates for a variety of compounds useful as agricultural chemicals or medicines.

Heretofore, various processes have been proposed to produce serine. Especially, as processes for producing serine from aziridine-2-carboxylic acid or its derivative, there have been known to treat lithium aziridine-2-carboxylate in 15% sulfuric acid [K. D. Gundermann, Chem. Ber., *93*, 1639 (1960)] and to treat isopropyl aziridine-2-carboxylate with perchloric acid [E. Kyburz, Helv. Chim. Acta, *49*, 359(1966)]. However, the former process requires extraordinarily excess sulfuric acid (about 12 times in molar ratio) compared with the starting lithium aziridine-2-carboxylate. In the former process, it is also necessary for the isolation of produced serine from the reaction system after the completion of the reaction to neutralize excess sulfuric acid with calcium hydroxide or barium hydroxide and then filter off the resultant calcium or barium sulfate. Thus, the former process is accompanied by a drawback that its process steps are complex and irksome. The former process also involves a problem that its reaction has an extremely low degree of volumetric efficiency. On the other hand, the latter process requires, subsequent to the reaction with perchloric acid, to drive off the reaction solvent through distillation and then to extract the reaction product with an alcohol. The latter process is thus not very easy to carry out in an industrial scale. Moreover, the thus-isolated serine still contains iso-serine, glycine, etc. Accordingly, the latter process requires a further purification step. This certainly renders the latter process very complex. Presence of halogen ions in the hydrolysis of aziridine-2-carboxylic acid or its derivative is disadvantageous, since it results in the formation of one or more by-products containing such halogen ions added thereto and, correspondingly, in lowered yield of DL-serine.

In addition, β-substituted-β-hydroxyamino acids may also be prepared from 3-substituted-aziridine-2-carboxylic acids or their derivatives. With respect to this preparation route, there have been known to treat a mixture of methyl 3-methylazaridine-2-carboxylate and ethyl 3-methylazirdine-2-carboxylate with perchloric acid and then to treat the resulting reaction product with hydrochloric acid so as to give threonine (Japanese Patent Laid-open No. 157555/1979) or to subject methyl 1-benzyl-3-phenylaziridine-2-carboxylate to ring-opening hydrolysis using perchloric acid and, thereafter, to reduce the resultant reaction product in the presence of a Pd/C catalyst so as to provide phenylserine. However, the former process is accompanied by such shortcoming that its steps are unavoidably complex and cumbersome, because it is indispensable, for the isolation of threonine from the resultant threonine-containing reaction mixture, to concentrate the reaction mixture, adjust its pH with aqueous ammonia and then purify the resulting threonine with an ion-exchange resin. Similarly, the latter process is very complex and irksome and is not considered to be a useful process from the industrial standpoint because it is required, after the perchloric acid treatment, to isolate N-benzylphenylserine and then subject it to reduction in the presence of the Pd/C catalyst in order to remove the benzyl group. Furthermore, the ring-opening hydrolysis reaction of a 3-substituted-aziridine-2-carboxylic acid or its derivative involves a potential danger that, if the reaction system contains any anions having nucleophilicity higher than that of water, for example, halogen ions, certain by-products coupled with such ions come out and the yield of the intended β-substituted-β-hydroxyamino acid becomes poor.

As has been mentioned above, the prior art preparation processes of DL-serine are accompanied by various difficulties, from not only their economical disadvantages but also their cumbersome operations,

and are not suited for practising them in an industrial scale. Furthermore, some processes have been proposed to prepare β-substituted-β-hydroxyamino acids. These proposals are however not fully satisfactory for their industrial applications. Thus, no satisfactory production process of β-hydroxyamino acids or their derivatives has yet been reported from the industrial viewpoint.

A number of the present inventors have previously invented a process for producing DL-serine, which comprises causing a strongly acidic ion-exchange resin to adsorb thereon aziridine-2-carboxylic acid, and then heating the thus-adsorbed aziridine-2-carboxylic acid in the presence of water (EP—A—0 034 0474). The present inventors have continued to develop the process, thus bringing about the present invention.

Disclosure of the Invention

An Object of this invention is to provide a process for preparing β-hydroxyamino acids, which process has been considerably simplified in terms of steps involved therein.

Another object of this invention is to provide a process for preparing β-hydroxyamino acids, which process is capable of providing the β-hydroxyamino acids with high yield without by-products.

A further object of this invention is to provide a process for preparing β-hydroxyamino acids, which process permits the isolation of the β-hydroxyamino acids through a simple treatment or operation.

In one aspect of this invention, there is thus provided the following process for preparing a β-hydroxyamino acid:

A process for preparing a β-hydroxyamino acid, which process comprises causing a strongly acidic ion-exchange resin to adsorb thereon aziridine-2-carboxylic acid derivative represented by the general formula (II):

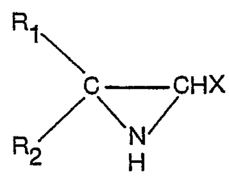

(II)

wherein $R_1$ represents a hydrogen atom, methyl group, a substituted or unsubstituted phenyl or pyridyl group, $R_2$ means a hydrogen atom or a methyl group, and X is —$CO_2H$, —$CO_2M$ (M: an alkali metal or alkaline earth metal), —$CO_2R_3$ ($R_3$: a lower alkyl group having 1—5 Carbon atoms or an aralkyl group), —$CONH_2$ or —CN, with the provision that in case that X is $CO_2H$ or $CO_2M$, then $R_1$ and $R_2$ are not both a hydrogen atom and then heating the thus-adsorbed aziridine-2-carboxylic acid derivative in the presence of water.

As specific examples of β-hydroxyamino acids to be produced in accordance with the process of this invention, may be mentioned DL-serine, threonine, β-hydroxyvaline, β-hydroxyphenylalanine, etc.

The present process was not known at all in the past. Compared with the above-described prior art processes, the present process has such merits that its steps are substantially simplified and it can obtain β-hydroxyamino acids with high yield, practically, without any by-products. The β-hydroxyamino acid, resulted from carrying out a reaction, can be isolated through such simple operations that it is only required to, subsequent to the heating step, treat the ion-exchange resin with aqueous ammonia to elute the β-hydroxyamino acid and then to concentrate the resultant eluate (i.e., washings obtained by the eluting) to dryness or to concentrate the resultant eluate and to allow the β-hydroxyamino acid to crystallize. It is also a valuable merit of the present process that, as mentioned above, the reaction, purification and isolation can be carried out as a series of continuous operations.

Since an aziridine-2-carboxylic acid derivative such as an ester, amide or nitrile of aziridine-2-carboxylic acid is adsorbed on an ion-exchange resin and then heated while still in the adsorbed state, the hydrolysis reaction of the ester, amide or nitrile moiety takes place concurrently with its ring-opening reaction and DL-serine is thus obtained immediately.

Where a β-substituted-aziridine-2-carboxylic acid or its salt is employed as a starting material, no iso-isomer is formed at all contrary to the case where unsubstituted aziridine-2-carboxylic acid or its salt is used. In addition, use of a β-substituted-aziridine-2-carboxylic acid derivative as a starting material permits the corresponding ring-opening reaction and hydrolysis reaction to proceed in a state adsorbed on an ion-exchange resin and the reaction product does not contain its iso-isomer.

Best Mode for Carrying Out the Invention

The aziridine-2-carboxylic acid or its derivative to be used in this invention is represented by the aforementioned general formula (II). More specifically, it may be an aziridine-2-carboxylic acid derivative represented by the general formula (III):

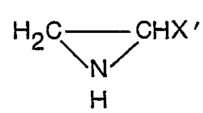

(III)

wherein X′ represents —CN, $CONH_2$ or —$CO_2R_3$($R_3$: a lower alkyl group having 1—5 carbon atoms or an

aralkyl group) — i.e., an ester, amide or nitrile of aziridine-2-carboxylic acid-; or a 3-substituted-aziridine-2-carboxylic acid or its derivative, i.e., its salt, ester, amide or nitrile, represented by the general formula (IV):

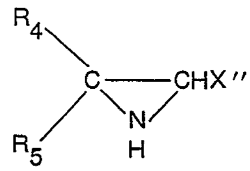

(IV)

wherein $R_4$ is a methyl group or a substituted or unsubstituted phenyl or pyridyl group, $R_5$ denotes a hydrogen atom or a methyl group, X'' means $-CO_2H$, $-CO_2M$ (M: an alkali metal or alkaline earth metal), $-CO_2R_3$ ($R_3$: a lower alkyl containing 1—5 carbon atoms or aralkyl group), $-CONH_2$ or $-CN$.

The above starting materials may be produced in accordance with a suitable process commonly known in the art, for example, by reacting their corresponding dihalogenopropionic acid derivatives or α-halogenoacrylic derivatives with ammonia [E. Kyburz, Helv. Chim. Acta., *49*, 368, (1966); Japanese Patent Laid-open No. 31850/1971].

Exemplary esters of aziridine-2-carboxylic acid may include the $C_1$—$C_5$ alkyl esters such as the methyl, ethyl, n-propyl, iso-propyl, n-butyl, iso-butyl, and s-butyl esters and the aralkyl esters such as the benzyl ester.

3-substituted-aziridine-2-carboxylic acids or their derivatives may be produced by suitable processes known per se in the art or their analogous processes, for example, by reacting β-substituted-α,β-dihalogenopropionic acids or their derivatives or β-substituted-α-halogenoacrylic acid derivatives with ammonia [Japanese Patent Laid-open No. 157555/1979; E. Kyburz, Helv. Chim. Acta, *49*, 359 (1966); G. Szeimies, Chem. Ber., *110*, 1792 (1977); E. P. Styhgach, Khim. Geterotsikl. Soedin, *1973*, 1523; Y. Yukawa, Mon. Ins. Sci. and Ind. Research, Osaka Univ., *14*, 191 (1957); E. P. Styngach, Izv. Akad. Nauk. Mold. SSR. Ser. Biol. Khim. Nauk., *1975*, 62)], through thermal decomposition reactions of 3-substituted-acrylic acid esters and azidocarboxylic acid esters (M. P. Samnes, J. Chem. Soc., Perkin Trans. 1, *1972*, 344), or, to obtain the alkali metal or alkaline earth metal salts of 3-substituted-aziridine-2-carboxylic acids, by treating esters of 3-substituted-aziridine-2-carboxylic acids with stoichiometrically equiamounts of alkali metal or alkaline earth metal hydroxides or by treating β-substituted-α-amino-β-halogenopropionic acids or their derivatives (esters, amides or nitriles) or β-substituted-α-halogeno-β-aminopropionic acids or their derivatives with alkali metal or alkaline earth metal hydroxides.

To carry out the process according to this invention, aziridine-2-carboxylic acid or its derivative represented by the general formula (II) is first of all adsorbed on a strongly acidic cation-exchange resin by causing an aqueous solution of aziridine-2-carboxylic acid or its derivative to flow through the cation-exchange resin or by adding the strongly acidic cation-exchange resin to the aqueous solution of aziridine-2-carboxylic acid or its derivative. Here, the aqueous solution of aziridine-2-carboxylic acid or its derivative may contain, without developing any problem or inconvenience, a water-miscible organic solvent such as methanol, ethanol, isopropyl alcohol or the like.

The strongly acidic cation-exchange resin to be used in the process of this invention may be of any type, for example, of the H-type, Na-type or $NH_4$-type. Generally speaking, it is however preferred to use an H-type one. The substrate (i.e., solid support) of the cation-exchange resin may be any one, including a gel-type, porous-type or macroporous-type substrate. The strongly acidic cation-exchange resin shall not be limited to any particular brand. It is feasible, without encountering any problems or inconvenience, to use two or more different brands of strongly acidic cation-exchange resins in combination. Where an alkali metal or alkaline earth metal salt of aziridine-2-carboxylic acid represented by the general formula (II) is used as a starting material, the ion-exchange resin may be employed in an amount, as an exchange capacity under wet and swollen conditions, of at least 1 equivalent weight, and preferably at least 1.2 equivalent weights per each equivalent weight of the total amount of aziridine-2-carboxylic acid and the metal ion forming the salt with aziridine-2-carboxylic acid. For examle, in the case of using 1 mole of the potassium salt of a 3-substituted-aziridine-2-carboxylic acid as a starting material and a strongly acidic cation-exchange resin having an overall exchange capacity of 2 equivalent weights per liter, it is necessary to use the cation-exchange resin in an amount of 1 liter or more, and preferably, 1.2 liters or more. Where aziridine-2-carboxylic acid or its derivative such as its ester, amide or nitrile represented by the general formula (II) is employed, the cation-exchange resin may be used in an amount of at least 1 equivalent weight; and preferably 1.2 equivalent weights or more as the exchange capacity under wet and swollen conditions per each equivalent weight of aziridine-2-carboxylic acid or its derivative per se. For example, in the case of using 1 mole of the ethyl ester of a 3-substituted-aziridine-2-carboxylic acid as a starting material and a strongly acidic cation-exchange resin having an overall exchange capacity of 2 equivalent weights per liter, the resin may be used in an amount of 0.5 liter or more, and preferably, 0.6 liter or more. Where the aqueous solution of a starting material contains substances susceptible to adsorption on a strongly acidic cation-exchange resin, including for example an inorganic salt such as sodium chloride or ammonium bromide or an amino group-containing compound, it is necessary to increase the amount of the cation-exchange resin by an amount at least equivalent to such substances.

According to the process of this invention, aziridine-2-carboxylic acid or its derivative represented by the general formula (II) is adsorbed on a strongly acidic cation-exchange resin by converting it into an aqueous solution, which may optionally contain a water-miscible organic solvent, for example, an alcohol such as methanol, ethanol or isopropanol, causing the thus-obtained solution to flow through a column packed with the cation-exchange resin and then washing the column with water or by adding the cation-exchange resin to the solution and then mixing them together. It is preferred to cause an aqueous solution of a starting material to flow through a column packed with a strongly acidic cation-exchange resin and then thoroughly washing the column with water so as to adsorb a 3-substituted-aziridine-2-carboxylic acid or its derivative on the cation-exchange resin, notably where the aqueous solution of the starting material contains any compound capable of forming through ion-exchange an acidic substance such as hydrochloric acid or hydrobromic acid, for example, sodium chloride or ammonium bromide since such an acidic substance serves to desorb the starting material from the cation-exchange resin or halogen ions may undergo a reaction with the starting material.

In the process of this invention, the strongly acidic cation-exchange resin carrying aziridine-2-carboxylic acid or its derivative represented by the general formula (II) adsorbed thereon is then heated in the presence of water. The heating may be effected in any suitable manner. However, it is necessary to keep the cation-exchange resin under wet and swollen state during the heating. This may be achieved, for example, by charging hot water continuously into a column packed with the cation-exchange resin or heating the column externally. Alternatively, the cation-exchange resin may be transferred to another vessel and then heated with stirring in the presence of water. The heating conditions are 1—100 hours at 40—120°C, and preferably 2—50 hours at 50—100°C. The reaction may still proceed at temperatures lower than 40°C, for example, at room temperature, but such low temperatures require considerably long time for the completion of the reaction and are thus impractical.

By heating aziridine-2-carboxylic acid or its derivative represented by the general formula (II) in an adsorbed state on the ion-exchange resin to have the starting material undergo a reaction there, a corresponding β-hydroxyamino acid is formed also in an adsorbed state on the same cation-exchange resin. In order to isolate the thus-adsorbed β-hydroxyamino acid, it is necessary to elute it from the ion-exchange resin by a method known per se in the art, for example, by eluting the thus-adsorbed β-hydroxyamino acid with aqueous ammonia, and evaporating the resultant eluate to dryness or concentrating the resultant eluate below the solubility of the β-hydroxyamino acid and then allowing it to crystallize.

The process of this invention will hereinafter be described with reference to the following examples. The 3-substituted-aziridine-2-carboxylates or 3-substituted-aziridine-2-carboxylic acid derivatives employed as starting materials in Examples 1—8, 10 and 11 were, each, a mixture of its cis-isomer and trans-isomers. Correspondingly, in these particular examples, the resultant β-hydroxyamino acids were each a mixture of its threo-isomer and erythro-isomer.

Incidentally, in the following examples, the purity and yield were determined by high-speed liquid chromatography and/or nuclear magnetic resonance (NMR).

Example 1

Through 60 ml of a strongly acidic cation-exchange resin, Lewatit® S—100(H-type) — product of Bayer A. G., was caused to flow 160 ml of an aqueous solution containing 12.9 g of ethyl 3-methylaziridine-2-carboxylate dissolved therein. It was then washed with 60 ml of distilled water to adsorb ethyl 3-methylaziridine-2-carboxylate thereon. Thereafter, the resultant cation-exchange resin was transferred into a 100 milliliter-flask, followed by a reaction at 80—85°C for 7 hours. Then, the cation-exchange resin was packed back in a column and subjected to elution with 90 ml of 5% aqueous ammonia and 60 ml of distilled water. The eluate was evaporated to dryness, thereby providing 11.4 g of threonine (purity: 92.5%; yield: 88.6%).

The above procedure was repeated using, as the starting material, a 66 cis-isomer: 34 trans-isomer mixture of ethyl 3-methylaziridine-2-carboxylate. Threonine was obtained as a 70 threo-isomer: 30 allo-isomer mixture.

Following the above procedure, threonine was prepared only from the cis-isomer of ethyl 3-methylaziridine-2-carboxylate. The resultant threonine contained, practically, its threo-isomer only.

Example 2

Through 60 ml of Lewatit® S—100(H-type) was caused to flow 136 ml of an aqueous solution containing 8.2 g of 3-methylaziridine-2-nitrile dissolved therein, followed by washing the cation-exchange resin with 60 ml of distilled water to adsorb 3-methylaziridine-2-nitrile thereon. Then, hot water of 80—85°C was allowed to circulate through the cation-exchange resin for 8 hours. After the completion of the reaction, the ion-exchange column was cooled and eluted with 90 ml of 5% aqueous ammonia and 60 ml of distilled water. The resulting eluate was evaporated to dryness and 7.6 g of a solid substance containing 4.9 g of threonine was obtained. The purity and yield of the thus-obtained threonine were 64.5% and 41.2% respectively.

Example 3

To 160 g of an aqueous solution containing 15.5 g of α-chloro-βamino-n-butylonitrile hydrochloride dissolved therein, was dropped little by little under stirring an aqueous solution obtained beforehand by

dissolving 12.8 g of sodium hydroxide in 90 g of water. The resultant reaction mixture was then heated to 60°C and allowed to undergo a reaction at 60—65°C for 6 hours.

The reaction mixture was cooled and then neutralized with a 5% aqueous sulfuric acid solution. It was then caused to pass through a column packed with 400 ml of Lewatit® S—100 (H-type). Then, distilled water was continuously flown through the column until no chlorine ions were detected in the washings from the column. Through the cation-exchange column on which 3-methylarizidine-2-carboxylic acid had been adsorbed, hot water of 85—90°C was circulated for 8 hours to carry out the reaction. After the completion of the reaction, the cation-exchange column was cooled, followed by an elution with 600 ml of 5% aqueous ammonia and 400 ml of distilled water. The resultant eluate was evaporated to dryness, thereby giving 11.0 g of threonine (purity: 88.7%; yield: 82%).

Example 4

The procedure of Example 3 was repeated except for the employment of 20.2 g of ethyl α-chloro-β-aminobutylate hydrochloride. Threonine was obtained in an amount of 10.5 g. Its purity and yield were 93.0% and 82.0% respectively.

Example 5

Through 30 ml of Lewatit® S—100 (H-type) was caused to flow 100 ml of an aqueous solution containing 7.2 g of isopropyl 3-methylaziridine-2-carboxylate, followed by washing it with 30 ml of distilled water to adsorb isopropyl 3-methylaziridine-2-carboxylate thereon. Thereafter, hot water of 80—85°C was circulated for 8 hours through the cation-exchange resin. After completion of the reaction, the ion-exchange column was cooled and then eluted with 45 ml of 5% aqueous ammonia and 30 ml of distilled water. The eluate was evaporated to dryness, resulting in 11.2 g of threonine (purity: 95.9%; yield: 90.3%).

Examples 6—8

The procedure of Example 5 was followed except for the replacement of each of the under-listed strongly acidic cation-exchange resin to Lewatit® S—100. Results are shown in Table 1.

TABLE 1

| Ex. No. | Strongly acidic cation-exchange resin | Threonine | |
|---|---|---|---|
| | | Purity (%) | Yield (%) |
| 6 | Lewatit SP—120 | 94.8 | 90.9 |
| 7 | Diaion SK—1B | 96.3 | 91.2 |
| 8 | Amberlite IR—121 | 93.5 | 89.6 |

Lewatit SP—120 is a trademark for a product of Bayer AG, Diaion SK—1B is a trademark for a product of Mitsubishi Chemical Industries, Ltd. and Amberlite IR—121 is a trademark for a product of Rohm & Haas Co.

Example 9

Through 30 ml of Lewatit® S—100 (H-type), was caused to pass 72 ml of an aqueous solution containing 5.8 g of 3,3-dimethylaziridine-2-carboxylic acid, followed by washing it with 30 ml of distilled water to adsorb 3,3-dimethyl-aziridine-2-carboxylic acid thereon. Thereafter, the cation-exchange resin was transferred into a 100 milliliter-flask and subjected there to a reaction at 80—85°C for 7 hours. It was then packed back into a column and eluted with 45 ml of 5% aqueous ammonia and 30 ml of distilled water. The resultant eluate was evaporated to dryness to give 6.4 g of β-hydroxyvaline (purity: 91.3%; yield: 88.5%).

Example 10

Through 40 ml of Lewatit® S—100 (H-type), was caused to pass 150 ml of an aqueous solution containing 10.3 g of isopropyl 3-phenylaziridine-2-carboxylate dissolved therein, followed by washing it with 40 ml of distilled water to adsorb isopropyl 3-phenylaziridine-2-carboxylate thereon. Then, hot water of 80—85°C was circulated through the cation-exchange resin for 8 hours. After completion of the reaction, the ion-exchange column was cooled and then eluted with 60 ml of 5% aqueous ammonia and 90 ml of distilled water. The resulting eluate was evaporated to dryness to obtain 9.0 g of a solid reaction product. The reaction product contained 83.3% of phenylserine (yield: 82.9%).

Example 11

Following the procedure of Example 10, 160 ml of an aqueous solution containing 8.1 g of 3-phenylaziridine-2-carboxylic acid amide was treated to give 8.7 g of a reaction product. It contained 56.3% of phenylserine (yield: 54.1%).

Example 12

One hundred sixty milliliters of an aqueous solution containing 10.3 g of isopropyl 3-(2-pyridyl)-aziridine-2-carboxylate were treated in much the same way as in Example 10 to give 9.2 g of a reaction product. It contained 80.4% of 2-pyridylserine (yield: 81.3%).

Example 13

6.8 g of aziridine-2-nitrile was dissolved in 100 ml of water and caused to flow through a column packed with 60 ml of a strongly acidic cation-exchange resin, Lewatit® S—100 (H-type)-product of Bayer AG, followed by washing it with 60 ml of distilled water to adsorb aziridine-2-nitrile thereon. Through the cation-exchange resin, hot water of 80—85°C was caused to circulate for 7 hours. After completion of the reaction, the ion-exchange column was cooled and then eluted with 90 ml of 5% aqueous ammonia and 120 ml of distilled water. The resultant eluate was concentrated to 9.0 g. The resulting solid substrate was collected through filtration and dried, thereby obtaining 3.5 g of DL-serine (purity: 99.0%; yield: 33.3%).

Example 14

Through 130 ml of a strongly acidic cation-exchange resin, Lewatit® S—100 (H-type)—product of Bayer A.G., was caused to pass 250 ml of an aqueous solution containing 20.2 g of methyl aziridine-2-carboxylate, followed by washing it with 130 ml of distilled water to adsorb methyl aziridine-2-carboxylate thereon. Then, the ion-exchange column was circulated with hot water of 90—95°C for 6 hours.

After completion of the reaction, the ion-exchange column was cooled and then eluted with 200 ml of 5% aqueous ammonia and 200 ml of distilled water. Upon evaporating the resultant eluate to dryness, 21.0 g of DL-serine was obtained (purity: 93.7%; yield: 93.8%).

Example 15

A reaction system of 27.4 g of isopropyl α,β-dibromopropionate and 350 ml of liquid ammonia was distilled to drive off ammonia. Its residue was dissolved in 300 ml of water and passed through 250 ml of Lewatit® S—100 (H-type). Distilled water was continuously passed through the column until no bromine ions were detected in the washings from the column. The ion-exchange resin column carrying isopropyl aziridine-2-carboxylate adsorbed thereon was thereafter circulated for 8 hours with hot water of 80—85°C. After completion of the reaction, the ion-exchange resin was cooled and then eluted with 400 ml of 5% aqueous ammonia and 250 ml of distilled water. The resulting eluate was concentrated to 17.0 g and the resulting solid substance was collected through filtration and then dried to give 5.5 g of DL-serine (purity: 97.5%; yield; 51.0%).

Example 16

Through 130 ml of Lewatit® S—100 (H-type) was caused to flow 170 ml of an aqueous solution containing 17.2 g of aziridine-2-carboxylic acid amide dissolved therein, followed by its washing with 130 ml of distilled water. The cation-exchange resin was then transferred into a 300 milliliter-flask and was allowed to undergo a reaction at 90—95°C for 7 hours. It was then packed back into a column and eluted with 190 ml of 5% aqueous ammonia and 130 ml of distilled water. The resultant eluate was concentrated to 26 g and the resulting solid substance was collected through filtration and then dried to give 7.9 g of DL-serine (purity: 98%; yield: 37.6%).

Example 17

Through 60 ml of Lewatit® S—100 (H-type), was caused to flow 160 ml of an aqueous solution containing 12.9 g of isopropyl aziridine-2-carboxylate dissolved therein, followed by washing it with 60 ml of distilled water to adsorb isopropyl aziridine-2-carboxylate thereon. The ion-exchange resin was then circulated for 8 hours with hot water of 85—90°C. After completion of the reaction, the ion-exchange column was cooled and then eluted with 90 ml of 5% aqueous ammonia and 60 ml of distilled water. Upon evaporating the resultant eluate to dryness, 10.0 g of DL-serine was obtained (purity: 92.3%; yield: 87.8%).

Examples 18—21

The procedure of Example 17 was repeated except for the substitution of each of the below-listed strongly acidic cation-exchange resins for Lewatit® S—100. Results are shown in Table 2.

TABLE 2

| Ex. No. | Strongly acidic cation-exchange resin | DL-serine | |
|---|---|---|---|
| | | Purity (%) | Yield (%) |
| 18 | Diaion SK—1B | 92.4 | 86.2 |
| 19 | Diaion PK—228 | 90.5 | 83.5 |
| 20 | Amberlite IR—121 | 94.3 | 90.3 |
| 21 | Lewatit SP—120 | 94.0 | 88.5 |

Diaion SK—1B and PK—228 are trademarks for products of Mitsubishi Chemical Industries, Ltd., Amberlite IR—121 is a trademark for a product of Rohm & Haas Co. and Lewatit SP—120 is a trademark for a product of Bayer AG.

Example 22

A reaction was carried out in much the same way as in Example 17. The resultant eluate was concentrated to 29.0 g and then allowed to develop a solid substance. The solid substance was collected through filtration and then dried to give 8.6 g of DL-serine (purity: 99.9%; yield: 81.7%).

**Claims**

1. A process for preparing a β-hydroxyamino acid, which process comprises causing a strongly acidic ion-exchange resin to adsorb thereon an aziridine-2-carboxylic acid derivative represented by the general formula (II):

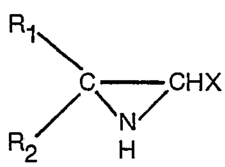

(II)

wherein $R_1$ represents a hydrogen atom, methyl group, or a substituted or unsubstituted phenyl or pyridyl group, $R_2$ means a hydrogen atom or a methyl group, and X is $—CO_2H$, $—CO_2M$ (M: an alkali metal or alkaline earth metal), $—CO_2R_3$ ($R_3$: a lower alkyl group having 1—5 carbon atoms or an aralkyl group), $—CONH_2$ or —CN, with the provision that in case that X is $CO_2H$ or $CO_2M$ $R_1$ and $R_2$ are not both a hydrogen atom; and then heating the thus-adsorbed aziridine-2-carboxylic acid derivative in the presence of water.

2. The process according to Claim 1, wherein the thus-adsorbed aziridine-2-carboxylic acid derivative having the general formula (II) is heated at 40—120°C.

3. The process according to Claim 1, wherein an aziridine-2-carboxylic acid derivative represented by the general formula (II), in which X is $—CO_2R_3$ or $—CONH_2$, or its derivative is adsorbed and heated.

4. The process according to Claim 1, wherein an aziridine-2-carboxylic acid derivative represented by the general formula (II), in which $R_1$ and $R_2$ mean either one of the following combinations: (methyl group; hydrogen atom), (methyl group; methyl group), (phenyl group; hydrogen atom), and (pyridyl group; hydrogen atom), or its derivative is adsorbed and heated.

5. A process according to Claim 2 for preparing DL-serine, wherein an aziridine-2-carboxylic acid derivative represented by the general formula (III):

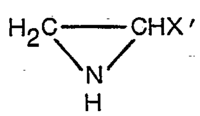

(III)

wherein X′ represents —CN, $—CONH_2$ or $—CO_2R_3$($R_3$: a lower alkyl group having 1—5 carbon atoms or an aralkyl group); is adsorbed and heated.

6. A process according to Claim 2 for preparing β-substituted-β-hydroxyamino acid, wherein a 3-substituted-aziridine-2-carboxylic acid or its derivative represented by the general formula (IV):

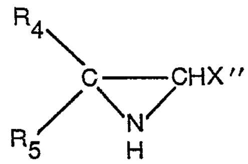
(IV)

wherein $R_4$ is a methyl group or a substituted or unsubstituted phenyl or pyridyl group, $R_5$ denotes a hydrogen atom or a methyl group, X'' means —$CO_2H$, —$CO_2M$ (M: an alkali metal or alkaline earth metal) or —$CO_2R_3$ ($R_3$: a lower alkyl containing 1—5 carbon atoms or an aralkyl group), —$CONH_2$ or —CN, is adsorbed and heated.

7. The process according to Claim 6, wherein 3-methylaziridine-2-carboxylic acid or its derivative is adsorbed and heated as the 3-substituted-aziridine-2-carboxylic acid represented by the general formula (IV) or its derivative to produce threonine.

**Patentansprüche**

1. Verfahren zur Herstellung einer β-Hydroxyaminosäure, das folgendes umfaßt:
Berwirken, daß an einem stark sauren Ionenaustauschharz ein Aziridin-2-carbonsäure-Derivat adsorbiert, das durch die allgemeine Formel (II) dargestellt wird:

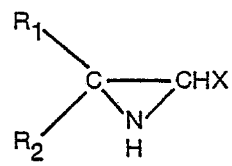
(II)

worin $R_1$ ein Wasserstoffatom, eine Methylgruppe oder eine substituierte oder nicht-substituierte Phenyl- oder Pyridylgruppe darstellt, $R_2$ ein Wasserstoffatom oder eine Methylgruppe bedeutet und X —$CO_2H$, —$CO_2M$ (M: ein Alkalimetall oder ein Erdalkalimetall), —$CO_2R_3$ ($R_3$: eine niedere Alkylgruppe mit 1 bis 5 Kohlenstoffatomen oder eine Aralkylgruppe), —$CONH_2$ oder —CN ist mit der Maßgabe, daß in dem Fall, daß X $CO_2H$ oder $CO_2M$ ist, $R_1$ und $R_2$ nicht beide ein Wasserstoffatom sind; und nachfolgendes Erhitzen des so adsorbierten Aziridin-2-carbonsäure-Derivats in Anwesenheit von Wasser.

2. Verfahren nach Anspruch 1, bei dem das so adsorbierte Aziridin-2-carbonsäure-Derivat mit der allgemeinen Formel (II) auf 40 bis 120°C erhitzt wird.

3. Verfahren nach Anspruch 1, bei dem ein Aziridin-2-carbonsäure-Derivat, das durch die allgemeine Formel (II), worin X —$CO_2R_3$ oder —$CONH_2$ ist, dargestellt wird oder sein Derivat adsorbiert und erhitzt wird.

4. Verfahren nach Anspruch 1, bei dem ein Aziridin-2-carbonsäure-Derivat, das durch die allgemeine Formel (II), worin $R_1$ und $R_2$ jeweils eine der folgenden Kombinationen beseichnen: (Methylgruppe, Wasserstoffatom), (Methylgruppe; Methylgruppe), (Phenylgruppe; Wasserstoffatom) und (Pyridylgruppe; Wasserstoffatom), dargestellt wird oder sein Derivat adsorbiert und erhitzt wird.

5. Verfahren nach Anspruch 2 zur Herstellung von DL-Serin, bei dem ein Aziridin-2-carbonsäure-Derivat, das durch die allgemeine Formel (III) dargestellt wird:

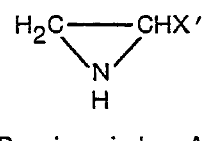
(III)

worin X' —CN, —$CONH_2$ oder —$CO_2R_3$ ($R_3$: eine niedere Alkylgruppe mit 1 bis 5 Kohlenstoffatomen oder eine Aralkylgruppe) darstellt; adsorbiert und erhitzt wird.

6. Verfahren nach Anspruch 2 zur Herstellung von β-substituierter-β-Hydroxyaminosäure, bei dem eine 3-substituierte-Aziridin-2-carbonsäure oder ihr Derivat, die durch die allgemeine Formel (IV) dargestellt werden:

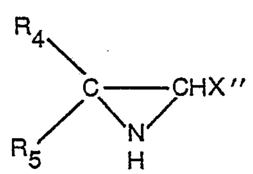
(IV)

worin $R_4$ eine Methylgruppe oder eine substituierte oder nicht-substituierte Phenyl- oder Pyridylgruppe ist,

$R_5$ ein Wasserstoffatom oder eine Methylgruppe bezeichnet, X'' —$CO_2H$, —$CO_2M$ (M: ein Alkalimetall oder ein Erdalkalimetall) oder —$CO_2R_3$ ($R_3$: eine niedere Alkylgruppe, die 1 bis 5 Kohlenstoffatome enthält, oder eine Aralkylgruppe), —$CONH_2$ oder —CN bezeichnet; adsorbiert und erhitzt wird.

7. Verfahren nach Anspruch 6, bei dem 3-Methylaziridin-2-carbonsäure oder ihr Derivat als die 3-substituierte-Aziridin-2-carbonsäure, die durch die allgemeine Formel (IV) dargestellt wird, oder ihr Derivat adsorbiert und erhitzt wird, um Threonin zu erzeugen.

**Revendications**

1. Procédé de préparation de β-hydroxyamino-acide, procédé qui consiste à faire adsorber sur une résine d'échange d'ions fortement acide un dérivé d'acide aziridine-2-carboxylique représenté par la formule générale (II):

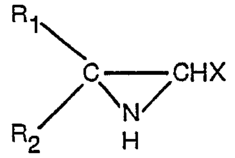

dans laquelle $R_1$ représente un atome d'hydrogène, un groupe méthyle, ou un groupe phényle ou pyridyle substitué ou non substitué, $R_2$ désigne un atome d'hydrogène ou un groupe méthyle, et X est —$CO_2H$, —$CO_2M$ (M: un métal alcalin ou un métal alcalino-terreux), —$CO_2R_3$ ($R_3$: un groupe alkyle inférieur ayant 1 à 5 atomes de carbone ou un groupe aralkyle), —$CONH_2$ ou —CN, à condition que, lorsque X est $CO_2H$ ou $CO_2M$, $R_1$ et $R_2$ ne soient pas tous deux un atome d'hydrogène; puis à chauffer le dérivé d'acide aziridine-2-carboxylique ainsi adsorbé en présence d'eau.

2. Procédé selon la revendication 1, dans lequel le dérivé d'acide aziridine-2-carboxylique ainsi adsorbé ayant la formule générale (II) est chauffé à 40—120°C.

3. Procédé selon la revendication 1, dans lequel un dérivé d'acide aziridine-2-carboxylique représenté par la formule générale (II), dans laquelle X est —$CO_2R_3$ ou —$CONH_2$, ou son dérivé, est adsorbé et chauffé.

4. Procédé selon la revendication 1, dans lequel un dérivé d'acide aziridine-2-carboxylique représenté par la formule générale (II), dans laquelle $R_1$ et $R_2$ l'une ou l'autre des combinaisons suivantes: (groupe méthyle; atome d'hydrogène), (groupe méthyle; groupe méthyle), groupe phényle; atome d'hydrogène), et (groupe pyridyle; atome d'hydrogène), ou son dérivé est adsorbé et chauffé.

5. Procédé selon la revendication 2, pour la préparation de DL-sérine, dans lequel un dérivé d'acide aziridine-2-carboxylique représenté par la formule générale (III):

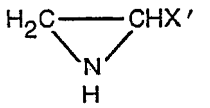

dans laquelle X' représente —CN, —$CONH_2$ ou —$CO_2R_3$ ($R_3$: un groupe alkyle inférieur ayant 1 à 5 atomes de carbone ou un groupe aralkyle); est adsorbé et chauffé.

6. Procédé selon la revendication 2, pour la préparation de β-hydroxyamino-acide substitué en β, dans lequel un acide aziridine-2-carboxylique substitué en 3 ou son dérivé représenté par la formule générale (IV):

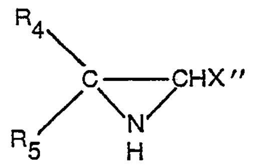

dans laquelle $R_4$ est un groupe méthyle ou un groupe phényle ou pyridyle substitué ou non substitué, $R_5$ désigne un atome d'hydrogène ou un groupe méthyle, X'' désigne —$CO_2H$, —$CO_2M$ (M: un métal alcalin ou un métal alcalino-terreux) ou —$CO_2R_3$ ($R_3$: un groupe alkyle inférieur ayant 1 à 5 atomes de carbone ou un groupe aralkyle), —$CONH_2$ ou —CN, est adsorbé et chauffé.

7. Procédé selon la revendication 6, dans lequel l'acide 3-méthylaziridine-2-carboxylique ou son dérivé est adsorbé et chauffé à titre d'acide aziridine-2-carboxylique substitué en 3 représenté par la formule générale (IV) ou de son dérivé pour produire de la thréonine.